# EUROPEAN PATENT APPLICATION

(11) **EP 2 540 258 A1**
(43) Date of publication of application: **02.01.2013**
(21) Application number: 10845992.6
(22) Date of filing: 22.02.2010
(51) Int. Cl.: A61F 2/84, A61F 2/82, A61F 2/06

(54) **DELIVERY SYSTEM FOR NEW SLIDE-AND-LOCK BIOABSORBABLE STENT**

(71) Applicant: Xinhua Hospital Affiliated Medical School Of Shanghai Jiaotong University, Shanghai 200092 (CN)
(72) Inventor: SUN, Kun, Shanghai 200092 (CN); SUN, Kang, Shanghai 200092 (CN); FENG, Qimao, Shanghai 200092 (CN); JIANG, Wenbo, Shanghai 200127 (CN); DOU, Hongjing, Shanghai 200127 (CN); LI, Wei, Shanghai 200127 (CN)
(74) Representative: Wilson, Justin Scott
(86) International application number: PCT/CN2010/070702
(87) International publication number: WO 2011/100870

(57) **Abstract**

A transcatheter delivery device for delivering a slide-and-lock stent to a stenosed lumen consists of an outer cannula (3), an inner sheath (2) and a balloon catheter (1). The distal end of the balloon catheter (1) is a balloon (5). The length and diameter of the balloon (5) can be chosen according to the requirement of the stent. A cone (4) is attached to the distal end of the balloon (5) to prevent the stent sliding to the distal end. The proximal part of the cone (4) can be inserted into the outer cannula (3) to maintain the external surfaces of the cone (4) and the outer cannula (3) on the same level. The inner sheath (2) is a little shorter than the balloon catheter (1) and extends to the proximal end of the balloon (5) so as to prevent the stent sliding to the proximal end.

## Description

### TECHNICAL FIELD

The present invention relates to a delivery device which is used to implant into the non-self-expandable slide-and-lock stent by the catheter across the cavity. The delivery device is used to expand stenosed points of blood vessel, trachea, esophagus, urethra, biliary tract, etc.

### BACKGROUND ART

In the field of interventional treatment, the stents are usually implanted into the stenotic peripheral position of blood vessel, trachea, esophagus, urethra, biliary tract to expand stenosed position, and to maintain lumen unimpeded effectively. The stents are guided into lumen under the state of non-expansion. When the stents are in stenotic position they will be expanded and fixed.

The traditionally stents can be devided into two types according to their expansion mechanism. One type is called self-expanding stent based on its own expansion. This kind of stent materials are mostly shape memory alloy and macromolecule polymer. The stent would be compressed into implantation device among internal and outer cannula in advance, then the implantation device is guided to the stenotic position. Finally the stent expands the stenosis position and maintains lumen unimpeded through withdrawing the outer cannula. Another type is balloon expandable stent which doesn't have the self-expansion characteristics. The stent would be previously pressed on the balloon surface, then the balloon reaches target lesion with guiding catheter leading. Finally the stent produces plastic deformation and sticks on the inner cavity to maintain lumen unimpeded through balloon expansion.

There is a new stent in addition to the traditional two types recently. This new slide-and-lock stent doesn't have shape memory property, and it presents as lamellar structure before dilation, rather than complete cylinder shape like traditional stent. Its expansion mechanism belongs to neither self-expanding nor balloon expandable after been implanted into lumen, so both of the delivery devices of self-expanding stent and balloon expandable stent are not suitable for the novel slide-and-lock stent.

### SUMMARY OF THE INVENTION

The objectives of this invention are to provide a delivery device for delivering the aforementioned new stent. The device combines the outer cannula and the balloon catheter, that result in stent not opening before dilation and accurate positioning, afterwards the stent expands sufficiently with the action of balloon expansion.

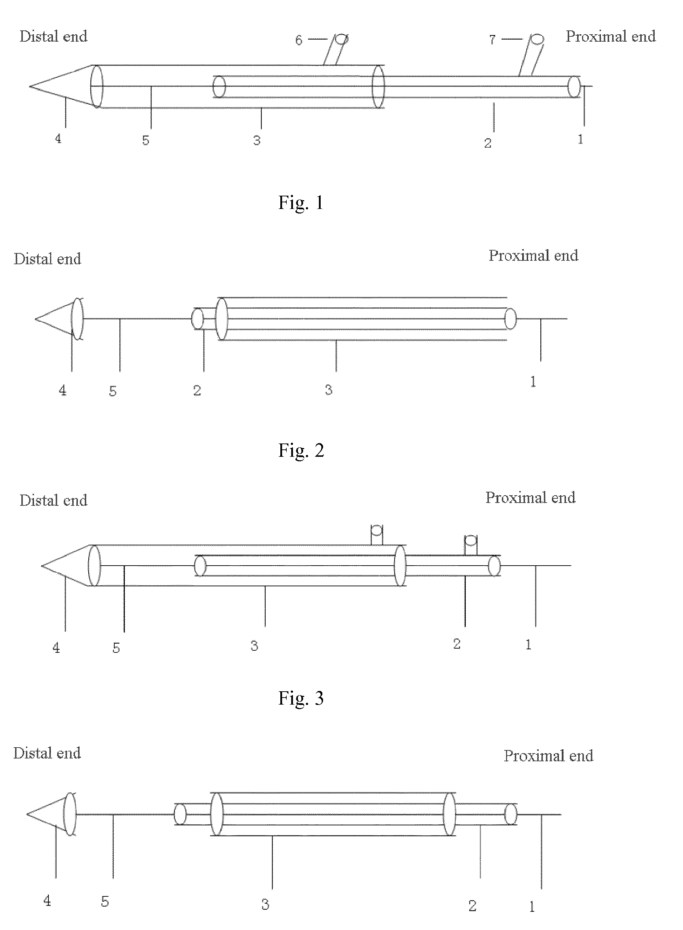

As shown in Fig.1, it is a delivery device of the new slide-and-lock stent in an embodiment. The delivery device consists of an outer cannula (3), an inner sheath (2) and a balloon catheter (1). The outer cannula (3) consists of a proximal end, a distal end and the cavity among the two ends. The inner sheath (2) also consists of a proximal end, a distal end and the cavity among the two ends. The external diameter of inner sheath (2) is proper to insert in the cavity of the outer cannula (3), and the length of the inner sheath (2) is slightly longer than that of the outer cannula (3). For instance, the length of the inner sheath (2) is 4-6cm longer than that of the outer cannula (3) in a mode of execution. The balloon catheter (1) also consists of a proximal end, a distal end and the cavity among the two ends. The external diameter of the balloon catheter (1) is proper to insert in the cavity of the inner sheath (2). The distal end of the balloon catheter (1) is a balloon (5). The length and diameter of the balloon (5) can be chosen according to the requirement of the stent. In a preferred embodiment, there is a metallic marker on each side of the balloon (5) which can position the stent. In a preferred embodiment, a cone (4) is attached to the distal end of the balloon (5) to prevent the stent sliding to the distal end. In an embodiment, the proximal part of the cone (4) can be inserted into the outer cannula (3) to maintain the external surfaces of the cone (4) and the outer cannula (3) on the same level. The inner sheath (2) is a little shorter than the balloon catheter (1) and extends to the proximal end of the balloon (5) so as to prevent the stent sliding to the proximal end. In the embodiment shown in Fig.1, the delivery device also includes two Y-type adapters, one is mounted on the proximal end of the inner sheath (2) and connects its cavity, and another is mounted on the proximal end of the outer cannula (3) and connects its cavity. Y-type adapters are applied to inject and remove needed liquid in the delivery procedure.

The stent is coiled on the surface of balloon (5) and fixed position between the cone (4) and the proximal end of the inner sheath (2), that could prevent the stent displacement. The stent cannot unfold as it is limited in the outer cannula (3). The delivery device equipped with stent is sent to stenosis vessel. When the delivery device is located destination accurately under the action of metallic marker of balloon (5), the outer cannula (3) is withdrawn. Then the balloon (5) is inflated to allow for the release of the stent, subsequently, the stent adheres closely to the vessel lining. Finally the outer cannula (3) and the inner sheath (2) are pulled out.

This invention also provides a complete set of equipment which includes a lamellar slide-and-lock stent and a stent delivery device. The delivery device consists of an outer cannula (3), an inner sheath (2) and a balloon catheter (1). The outer cannula (3) consists of a proximal end, a distal end and the cavity among the two ends. The inner sheath (2) also consists of a proximal end, a distal end and the cavity among the two ends. The external diameter of inner sheath (2) is proper to insert in the cavity of the outer cannula (3), and the length of the inner sheath (2) is slightly longer than that of the outer cannula (3). For instance, the length of the inner sheath (2) is 4-6cm longer than that of the outer cannula (3) in a mode of execution. The balloon catheter (1) also consists of a proximal end, a distal end and the cavity among the two ends. The external diameter of the balloon catheter (1) is proper to insert in the cavity of the inner sheath (2). The distal end of the balloon catheter (1) is a balloon (5). The length and diameter of the balloon (5) can be chosen according to the requirement of the stent. In a preferred embodiment, there is a metallic marker on each side of the balloon (5) which can position the stent. In a preferred embodiment, a cone (4) is attached to the distal end of the balloon (5) to prevent the stent sliding to the distal end. In an embodiment, the proximal part of the cone (4) can be inserted into the outer cannula (3) to maintain the external surfaces of the cone (4) and the outer cannula (3) on the same level. The inner sheath (2) is a little shorter than the balloon catheter (1) and extends to the proximal end of the balloon (5) so as to prevent the stent sliding to the proximal end. In the embodiment shown in Fig.1, the delivery device also includes two Y-type adapters, one is mounted on the proximal end of the inner sheath (2) and connects its cavity, and another is mounted on the proximal end of the outer cannula (3) and connects its cavity. Y-type adapters are applied to inject and remove needed liquid in the delivery procedure. The lamellar integration slide-and-lock biological stent comprises the following structures: an applanate stent body with mesh structure; the stent head which is located at one end of said stent body, said stent head is integrated with said stent body with matched size, and which provides a slide-and-lock effect when the stent is being curled; stent clips, which are integrated with the stent body and fix the tubular structure of the stent when it is being curled.

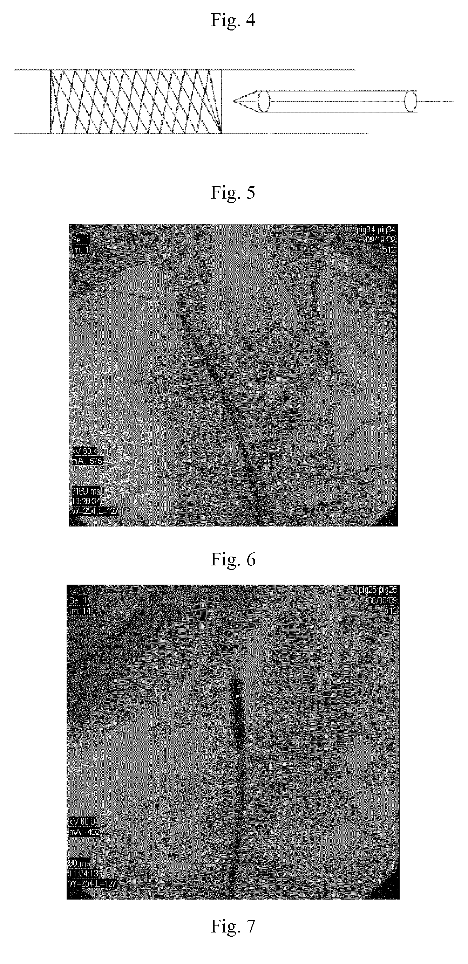

Next, the delivery procedure of delivery device will be described in detail:
1. As shown in Fig.2, the balloon (5) is sucked into negative pressure by force pump from the proximal end of the balloon catheter (1), the distal end of the balloon (5) and the inner sheath (2) are exposed when the outer cannula (3) is withdrawn.
2. As shown in Fig.3, the stent is coiled on the surface of balloon (5); the stent and cone (4) are enclosed by the outer cannula (3) when the outer cannula (3) is pushed to the distal end.
3. As shown in Fig.4, the delivery device mounted with stent is sent to target lesion by following a guide wire fed. When the stent is in position by the metallic marker orientation, the outer cannula (3) is withdrawn in order to expose the stent. Then the balloon (5) is inflated by force pump pressuring to allow for the release of the stent.
4. As shown in Fig.5, after the stent expands completely, the delivery device is withdrawn and the stent is left in vessel to support the vessel wall.

### BRIEF DESCRIPTION OF THE DRAWINGS

Fig.1 is a schematic view of the stent delivery device according to an embodiment of the present invention. 1, balloon catheter; 2, inner sheath; 3, outer cannula; 4, cone; 5, balloon; 6, Y-type adapter; 7, Y-type adapter.
Fig.2 is a schematic view of the exposed balloon and the distal end of the inner sheath when the outer cannula is withdrawn before implantation procedure. 1, balloon catheter; 2, inner sheath; 3, outer cannula; 4, cone; 5, balloon.
Fig.3 is a schematic view of that the stent is mounted on the balloon; the stent is enclosed by the outer cannula when the outer cannula is pushed to the distal end. 1, balloon catheter; 2, inner sheath; 3, outer cannula; 4, cone; 5, balloon (the stent is mounted on it).
Fig.4 is a schematic view of that the stent is exposed when the outer cannula is withdrawn from the proximal end. 1, balloon catheter; 2, inner sheath; 3, outer cannula; 4, cone; 5, balloon (the stent is mounted on it).
Fig.5 is a schematic view of that after the stent expands completely, the delivery device is withdrawn by following a guide wire fed and the stent is left in vessel.
Fig.6 is the angiography of that the stent is implanted to the left iliac artery of pig by the delivery device, when the stent is positioned at target site, the outer cannula is withdrawn from the proximal end of the balloon catheter.
Fig.7 is the angiography of that the stent is released along with balloon dilation by force pump pressuring.
Fig.8 is the angiography of that after the stent releases completely, the delivery device is withdrawn and the vessel with stent keeps flowing freely.

### DETAILED DESCRIPTION OF THE PREFERRED EMBODIMENTS

### Example 1

In the simulation vessel in vitro, the slide-and-lock polycaprolactone (PCL) stent with small barbs on each side is delivered by this special delivery device, the result of stent implantation and releasing is measured.

### 1. Materials and methods:

Materials: ten slide-and-lock polycaprolactone (PCL) stents of 20x8 mm, rubber hose of 6 cm diameter, stent delivery device and force pump.

### Methods:

(1) The balloon of the delivery device is sucked into negative pressure by the force pump; the stent is coiled on the surface of the balloon when the outer cannula is withdrawn. Then the stent is enclosed when the outer cannula is pushed to the cone portion.
(2) The stent delivery device is delivered to the artificial blood vessel target by following a guide wire fed; the stent is dilated and released at 12 atmospheres for 30s.
(3) The balloon is sucked into negative pressure and after that the balloon is withdrawn.

### 2. Performance index

(1) Success rate of stent implantation, evaluation criterion: success to target site, stent not falling off and shifting, position accurately.
(2) Complication rate: whether stent shift or fall off, balloon damage or lancination, vessel damage or lancination.
(3) Releasing pressure: the pressure released by the stent with expanding completely.

### 3. Result

The stent is released from delivery device under normal pressure (10-14 atm). The stent doesn't shift and fall off, the vessel and the balloon don't damage and lancination. The stent is delivered to target site successfully and positioned accurately.

**Table 1: the result of slide-and-lock PCL stents implantation**

| | n | Releasing pressure (atm) | Complication rate (%) | Success rate of implantation (%) |
|---|---|---|---|---|
| Delivery device | 10 | 12±2 | 0 | 100 |

### Example 2

In the simulation vessel in vitro, the slide-and-lock polydioxanone (PDO) stent with small barbs on each side is delivered by this special delivery device, the result of stent implantation and releasing is measured.

### 1. Materials and methods:

Materials: ten slide-and-lock polydioxanone (PDO) stents of 20x8 mm, rubber hose of 6 cm diameter, stent delivery device and force pump.

### Methods:

(1) The balloon of the delivery device is sucked into negative pressure by the force pump; the stent is coiled on the surface of the balloon when the outer cannula is withdrawn. Then the stent is enclosed when the outer cannula is pushed to the cone portion.
(2) The stent delivery device is delivered to the artificial blood vessel target by following a guide wire fed; the stent is dilated and released at 12 atmospheres for 30s.
(3) The balloon is sucked into negative pressure and after that the balloon is withdrawn.

### 2. Performance index

(1) Success rate of stent implantation, evaluation criterion: success to target site, stent not falling off and shift, position accurately.
(2) Complication rate: whether stent shift or fall off, balloon damage or lancination, vessel damage or lancination.
(3) Releasing pressure: the pressure released by the stent with expanding completely.

### 3. Result

The stent is released from delivery device under normal pressure (11-15 atm). The stent doesn't shift and fall off, the vessel and the balloon don't damage and lancination. The stent is delivered to target site successfully and positioned accurately.

**Table 2: the result of slide-and-lock PCL stents implantation**

| | n | Releasing pressure (atm) | Complication rate (%) | Success rate of implantation (%) |
|---|---|---|---|---|
| Delivery device | 10 | 13±2 | 0 | 100 |

### Example 3

In the iliac artery vessel of pig, the slide-and-lock polycaprolactone (PCL) stent with small barbs on each side is delivered by this special delivery device, the result of stent implantation and releasing is measured.

### 1. Materials and methods:

Materials: ten pigs weighting 25 kg, puncture needle, guide wire fed, 7F sheath, omnipaque, ketamine, heparin, stent delivery device, force pump, GE-2005 angiogram.
Sample content: ten pigs, fifteen slide-and-lock PCL stents of 20x8 mm.
Methods: (Fg.6, Fg.7, Fg.8)
(1) After each pig is anesthetized by 10mg/kg ketamine, tracheal intubate, ECG monitoring, covered with sterile drape, puncture left carotid artery, implant 7F sheath.
(2) Angiography of the left and right carotid artery is performed; the diameter of the stent is 25% bigger than that of the vessel.
(3) The balloon of the delivery device is sucked into negative pressure by the force pump; the stent is coiled on the surface of the balloon when the outer cannula is withdrawn. Then the stent is enclosed when the outer cannula is pushed to the cone portion. The stent delivery device is delivered to the target portion by following a guide wire fed, the stent is dilated and released at 12-15 atmospheres for 30s. The dosage of heparin is 100U/kg.
(4) The balloon is sucked into negative pressure and after that the balloon is withdrawn. Angiography is performed once again.

### 2. Performance index

(1) Success rate of stent implantation, evaluation criterion: success to target site, stent not falling off, position accurately under DSA.
(2) Complication rate: whether stent shift or fall off, balloon damage or lancination, vessel damage or lancination, pig die, etc.

### 3. Result

The stent is delivered to the target site successfully by the delivery device. The stent doesn't shift and fall off. The stent is released and positioned accurately from delivery device under X-ray. The stent is expanded successfully and the vessel with stent flow freely. The balloon and the vessel are not damaged.

**Table 3: the result of slide-and-lock PCL stents implantation**

| | n | Releasing pressure (atm) | Complication rate (%) | Success rate of implantation (%) |
|---|---|---|---|---|
| Delivery device | 15 | 12±2 | 0 | 100 |

### Example 4

In the iliac artery vessel of pig, the slide-and-lock polydioxanone (PDO) stent with small barbs on each side is delivered by this special delivery device, the result of stent implantation and releasing is measured.

### 1. Materials and methods:

Materials: ten pigs weighting 25kg, puncture needle, guide wire fed, 7F sheath, omnipaque, ketamine, heparin, stent delivery device, force pump, GE-2005 angiogram.
Sample content: ten pigs, fifteen slide-and-lock PDO stents of 20x8 mm.
Methods: (Fg.6, Fg.7, Fg.8)
(1) After each pig is anesthetized by 10mg/kg ketamine, tracheal intubate, ECG monitoring, covered with sterile drape, puncture left carotid artery, implant 7F sheath.
(2) Angiography of the left and right carotid artery is performed; the diameter of the stent is 25% bigger than that of the vessel.
(3) The balloon of delivery device is sucked into negative pressure by the force pump; the stent is coiled on the surface of the balloon when the outer cannula is withdrawn. Then the stent is enclosed when the outer cannula is pushed to the cone portion. The stent delivery device is delivered to the target portion by following a guide wire fed, the stent is dilated and released at 12-15 atmospheres for 30s. The dosage of heparin is 100U/kg.
(4) The balloon is sucked into negative pressure and after that the balloon is withdrawn. Angiography is performed once again.

### 2. Performance index

(1) Success rate of stent implantation, evaluation criterion: success to target site, stent not fall off, position accurately under DSA.
(2) Complication rate: whether stent shift or fall off, balloon damage or lancination, vessel damage or lancination, pig die, etc.

### 3. Result

The stent is delivered to the target site successfully by the delivery device. The stent doesn't shift and fall off. The stent is released and positioned accurately from delivery device under X-ray. The stent is expanded successfully and the vessel with stent flow freely. The balloon and the vessel are not damaged.

**Table 4: the result of slide-and-lock PCL stents implantation**

| | n | Releasing pressure (atm) | Complication rate (%) | Success rate of implantation (%) |
|---|---|---|---|---|
| Delivery device | 15 | 11±3 | 0 | 100 |

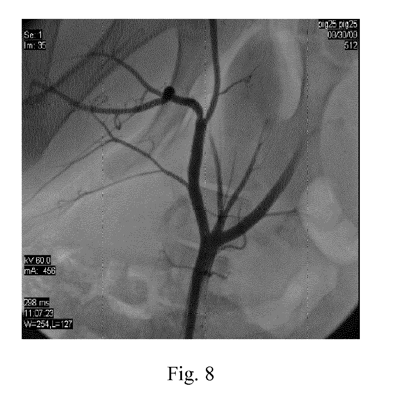

Fig.6 is the angiography of that the stent is implanted to the left iliac artery of pig by the delivery device, when the stent is positioned at the target site, the outer cannula is withdrawn from the proximal end of the balloon catheter. Fig.7 is the angiography of that the stent is released along with balloon dilation by force pump pressuring. Fig.8 is the angiography of that after the stent releases completely, the delivery device is withdrawn and the vessel with stent keeps flowing freely.

The patent, patent application, publication, file said in this invention don't admit that any of aforementioned documents belong to existing technology, and also don't admit the content or date of aforementioned documents.

Any improvements people make in preceding text couldn't be independent of the basic content. Although this invention has been disclosed in the context of several embodiments in detail, it will be understood by those skilled in the art that any improvements still fall within the scope of the invention. This narrative described invention can be implemented under the conditions of lack of one or more elements. Accordingly, the expressions "contain" "mainly consist of" "consist of" can be substituted for one another in the examples of the invention. Accordingly, the terminology and representation said above are used to describe content of the invention and not to limit scope of the invention. It will be understood that the present invention extends beyond the specifically disclosed embodiments to other alternative embodiments and/or uses of the invention and obvious modifications and equivalents thereof.

## Claims

1. A delivery device for delivering a lamellar slide-and-lock biological stent to a stenosed lumen comprising:
an outer cannula, consisting of a proximal end, a distal end and the cavity among the two ends,
an inner sheath, consisting of a proximal end, a distal end and the cavity among the two ends, the external diameter of said inner sheath is proper to insert in the cavity of said outer cannula,
a balloon catheter, consisting of a proximal end, a distal end and the cavity among the two ends, the external diameter of said balloon catheter is proper to insert in the cavity of said inner sheath, there is a balloon in the distal end of said balloon catheter, the lamellar slide-and-lock biological stent can coil on the surface of said balloon and subsequently fixed on the stenosed lumen through the catheter.

2. The delivery device of claim 1, wherein said lamellar slide-and-lock biological stent comprising:
an applanate stent body with mesh structure,
the stent head, which is located at one end of said stent body, said stent head is integrated with said stent body with matched size, and which provides a slide-and-lock effect when the stent is being curled,
stent clips, which are integrated with the stent body and fix the tubular structure of the stent when it is being curled.

3. The delivery device of claim 1, wherein the length of said inner sheath is longer than that of said outer cannula but slightly shorter than that of said balloon catheter, said inner sheath can reach the proximal end of the balloon of said balloon catheter, which can prevent the stent sliding to the proximal end..

4. The delivery device of claim 1, wherein a cone is attached to the distal end of the balloon to prevent the stent sliding to the distal end.

5. The delivery device of claim 4, wherein the proximal part of the cone can be inserted into the outer cannula to maintain the external surfaces of the cone and the outer cannula on the same level.

6. The delivery device of claim 1, wherein there is a metallic marker on each side of said balloon which can position the stent.

7. The delivery device of claim 1, wherein the delivery device also includes two Y-type adapters, one is mounted on the proximal end of the inner sheath and connects its cavity, another is mounted on the proximal end of said outer cannula and connects its cavity.

8. A complete set of equipment, comprising:
said stent delivery device of claim 1,
and the lamellar slide-and-lock stent, comprising:
an applanate stent body with mesh structure,
the stent head, which is located at one end of said stent body, said stent head is integrated with said stent body with matched size, and which provides a slide-and-lock effect when the stent is being curled,
stent clips, which are integrated with the stent body and fix the tubular structure of the stent when it is being curled.
